# EUROPÄISCHE PATENTSCHRIFT

(11) **EP 2 574 277 B1**
(45) Veröffentlichungstag und Bekanntmachung des Hinweises auf die Patenterteilung: **10.04.2019**
(21) Anmeldenummer: 12006706.1
(22) Anmeldetag: 26.09.2012
(51) Int. Cl.: A61B 5/022

(54) **Wickelmanschette für das Messen des Blutdrucks**
Compression sleeve for the measurement of blood pressure
Brassard enroulé pour la mesure de la pression sanguine

(30) Priorität: 28.09.2011 DE 102011115186
(43) Veröffentlichungstag der Anmeldung: 03.04.2013
(73) Patentinhaber: Paul Hartmann AG, 89522 Heidenheim (DE)
(72) Erfinder: Wegner, Ronny, 89233 Neu-Ulm (DE); Schmid, Uwe, 89558 Steinenkirch (DE); Miho, Stevan, 89522 Heidenheim (DE); Held, Fred, 22299 Hamburg (Vertrag) (DE)

(56) Entgegenhaltungen:
- WO-A1-2012/084190
- DE-A1-102007 037 770
- DE-A1-102010 056 241
- JP-A- 2004 195 056
- JP-U- 62 078 904
- JP-U- 62 078 905
- US-A1- 2005 251 052
- US-A1- 2007 106 166
- PAUL HARTMANN AG ED - PAUL HARTMANN AG: "Tensoval duo control - Gebrauchsanleitung", INTERNET CITATION, 1. Juli 2011 (2011-07-01), Seiten 1-17, XP002673591, Gefunden im Internet: URL:http://www.tensoval.de/fileadmin/data/ de-DE/pdfs/Gebrauchsanweisung_TDC_II.pdf [gefunden am 2012-04-10]

## Beschreibung

Die vorliegende Erfindung betrifft eine Wickelmanschette für das Messen des Blutdrucks am Oberarm eines Menschen.

Im Stand der Technik sind zwei unterschiedliche Arten von Manschetten für die Messung des Blutdrucks bekannt: Zugbügelmanschetten und Wickelmanschetten. Beide Manschettentypen weisen üblicherweise einen ersten Längsabschnitt mit einer expandierbaren Blase sowie einen Endabschnitt auf. Der wesentliche Unterschied zwischen beiden Manschettentypen besteht in der Art, wie beim Anlegen der Manschette der Endabschnitt auf dem ersten Längsabschnitt festgelegt werden kann, um einen Hohlzylinder zu bilden, welcher den Arm des Benutzers aufnehmen kann.

Zugbügelmanschetten zeichnen sich dadurch aus, dass der Endabschnitt durch ein am Ende des ersten Längsabschnitts angeordnetes Schlaufenelement hindurch geführt werden kann. Dann kann der Endabschnitt auf den ersten Längsabschnitt zurück umgeschlagen werden. Es erfolgt also beim Anlegen der Zugbügelmanschette ein die Wickelrichtung ändernder Umschlag des Endabschnitts.

Die Außenseite des Endabschnitts ist dann auf der Außenseite des ersten Längsabschnitts wiederablösbar festlegbar. Eine solche Zugbügelmanschette ist beispielsweise in der DE102010056241 beschrieben.

Im Gegensatz zu Zugbügelmanschetten wird bei Wickelmanschetten der Endabschnitt ohne Umkehrung der Wickelrichtung aufgewickelt. Solchenfalls kann die Innenseite des Endabschnitts auf der Außenseite des ersten Längsabschnitts wiederablösbar festgelegt werden.

Um eine derartige Wickelmanschette anzulegen, muss ein Benutzer zunächst den ersten Längsabschnitt der Manschette auf den Oberarm auflegen. Schließt der Benutzer die Manschette durch wickeln des Endabschnitts um die Außenseite des ersten Längsabschnitts, bildet die Manschette aus im Wesentlichem dem ersten Längsabschnitt einen Körper in Form eines Zylindermantels aus, wobei sich im Inneren des Zylindermantels ein Hohlraum befindet. Dieser Hohlraum kann den Oberarm des Benutzers aufnehmen. Der Innenumfang des Zylindermantels bzw. der angelegten Manschette wird dann durch Zug an dem Endabschnitt auf den Umfang des Oberarms des Benutzers angepasst. Der Endabschnitt kann dann wiederablösbar auf dem ersten Längsabschnitt festgelegt werden. Weiterhin umfasst der erste Längsabschnitt einen ersten unteren Randbereich sowie einen ersten oberen Randbereich.

Derartige Wickelmanschetten umfassen außerdem eine expandierbare Blase, die vorzugsweise über einen Druckschlauch mit einem Gerät zum Einleiten eines Fluids in die Blase verbindbar ist. Für eine zuverlässige Messung des Blutdrucks ist es besonders wichtig, dass die Blase im angelegten Zustand der Manschette den Oberarmbereich des Benutzers derart umschließt, dass beim Einleiten des Fluids eine Expansion der Blase die im Bereich des am Bizeps befindlichen Blutgefäße (Arteria brachialis) zuverlässig komprimiert. Da diese korrekte Positionierung der Blase für den Benutzer während des Anlegens der Manschette nicht einfach zu erkennen ist, ist bei zahlreichen im Markt befindlichen Manschetten der Druckschlauch bevorzugt auf der Außenseite des ersten Längsabschnitts, insbesondere bevorzugt im unteren Bereich des ersten Längsabschnitts angeordnet.

Auf diese Weise ist der Druckschlauch im angelegten Zustand der Manschette auf dem Bizeps des Benutzers oberhalb der Ellenbeuge positioniert. Diese Anordnung des Druckschlauchs zeigt dem Benutzer an, dass die Blase den entsprechenden Teil des Oberarms im Bereich des Bizeps derart umschließt, dass beim Einleiten des Fluids eine Expansion der Blase die am Bizeps befindliche Arteria brachialis zuverlässig abdrückt. Manschetten dieses Typs sind seit langem bekannt. Dem Stand der Technik entsprechende Messmanschetten sind z.B. in den Patent- bzw Gebrauchsmusterdokumenten US2005/251052A1 (10.11.2005), US2007/106166A1 (10.05.2007), JP62078904U (20.05.1987) und JP62078905U (20.05.1987) offenbart.

Für eine korrekte Messung des Blutdrucks eines menschlichen Benutzers ist es essentiell, dass die Manschette, insbesondere deren Blase, korrekt am Oberarm des Benutzers positioniert ist. Da ein Benutzer während des Anlegens der Manschette an dem Endabschnitt der Manschette zieht, um den Umfang der Manschette an den Umfang des Oberarms anzupassen, kommt es häufig vor, dass die Manschette in Längsrichtung, welche der Umfangsrichtung der an den Benutzer angelegten Manschette entspricht, auf dem Oberarm des Benutzers verrutscht. Dies hat eine unvorteilhafte Positionierung der Blase zur Folge, was zu einer ungenauen Messung des Blutdrucks führt. Der Benutzer ist daher gezwungen, die Position der Manschette auf dem Oberarm nachzukorrigieren. Diese umständliche Korrektur durch den Benutzer der Manschette kann zwar das Verrutschen der Manschette beim Anlegen ausgleichen, ist jedoch, insbesondere bei Anwendung der Manschette durch nicht dafür geschulte Personen, oft ungenau. Dieses Problem der genauen und einfach handhabbaren Positionierung der Manschette auf dem Oberarm des Benutzers stellt bei derzeitigem Stand der Technik ein ungelöstes Problem dar.

Der vorliegenden Erfindung liegt daher die Aufgabe zugrunde, eine Manschette für das Messen des Blutdrucks anzugeben, die die genannten Nachteile des Standes der Technik vermeidet und letzteren in vorteilhafter Weise weiterentwickelt. Insbesondere soll das Anlegen und die Positionierung der Manschette vereinfacht werden.

Zur Lösung dieser Aufgabe schlägt die Erfindung eine Wickelmanschette für das Messen des Blutdrucks gemäß Anspruch 1 vor. Dabei umfasst die Manschette an einem ersten unteren Randbereich eines ersten Längsabschnitts eine Positionierhilfe, wobei die Positionierhilfe eine Aussparung aufweist. Als ein unterer Randbereich wird im Rahmen dieser Erfindung derjenige Randbereich der Manschette verstanden, der im angelegten Zustand der Manschette in Richtung Ellenbeuge weist. Ein oberer Randbereich ist nach diesem Verständnis ein Randbereich, der in Richtung der Schulter weist. Diese Positionierhilfe ist dadurch vorteilhaft, dass die Aussparung so am Innenbereich der Ellenbeuge des Benutzers anlegbar ist, dass die Gefahr eines Verrutschens der Manschette auf dem Arm in Längsrichtung vermindert wird. Auf diese Weise ist die Blase in vorteilhafter Weise so positioniert, dass eine genaue und verlässlich reproduzierbare Messung des Blutdrucks gewährleistet ist. Der Begriff "Blase" bezeichnet dabei jede Art von Hohlkörper, der einen oder mehrere Hohlräume oder eine schwammartige Struktur aufweist und dafür geeignet ist, bei Befüllung mit einem Fluid zu expandieren. Die Blase ist in einem Blasenbereich der Manschette angeordnet, wobei sich der Blasenbereich in Längsrichtung vorzugsweise in etwa ausgehend von dem Endabschnitt bis zu dem Ende des ersten Längsabschnitts erstreckt.

Die Blase kann von einer die Innenseite (im angelegten Zustand die körperzugewandte Seite) und einer die Außenseite (im angelegten Zustand die körperabgewandte Seite) der Wickelmanschette bildenden Materiallagen selbst gebildet werden, wobei diese Materiallagen solchenfalls bereichsweise, das heißt dort, wo die Materiallagen die Blase bilden, zugleich den Blasenbereich bilden und begrenzen. Die Blase kann auch in einer alternativen Ausführungsform ein separates Bauteil darstellen, das zwischen diesen Materiallagen angeordnet und dort aufgenommen ist. Denkbar und vorteilhaft wäre es nach einer weiteren Ausführungsform, wenn die Blase, vorzugsweise allseitig, von einer separaten Komponente, insbesondere von einer Hülle oder Tasche umgeben oder aufgenommen ist, die Ihrerseits zwischen eine die Innenseite und eine die Außenseite der Wickelmanschette bildenden Materiallagen vorgesehen ist.

Die Blase hat vorzugsweise eine Länge in Längsrichtung von mindestens ca. 80% und maximal 100% des Oberarmumfangs eines Benutzers. Vorzugsweise beträgt die Länge der Blase, also deren maximale Erstreckung in der Längsrichtung, 10 cm bis 40 cm, insbesondere 22 cm bis 25 cm. Des Weiteren ist es bevorzugt, wenn die Breite der Blase, also deren maximale Erstreckung in der Querrichtung 8 cm bis 16 cm, insbesondere 11,5 cm bis 13,5 cm beträgt. Die hier angegebenen Abmessungen sind besonders für Manschetten in mittlerer und großer Größe geeignet. Denkbar und vorteilhaft ist es aber auch weitere, entsprechend angepasste Blasenabmessungen für noch kleinere oder noch größere Varianten von Manschetten vorzusehen.

Vorzugsweise weist die Manschette in an sich üblicher Weise einen Druckschlauch auf oder die Manschette ist an einen Druckschlauch anschließbar, welcher mit einem Gerät zum Einleiten eines Fluids in die Blase verbindbar ist. Ein Druckschlauch ist eine Struktur aus einer oder mehreren Leitungen, die dafür geeignet ist, ein Fluid, wie ein Gas oder eine Flüssigkeit, unter Druck zu leiten und der Blase zum Zwecke der Expansion der Blase zuzuführen.

Der die Blase enthaltende Blasenbereich weist sich vorzugsweise in der Längsrichtung erstreckende untere und obere Blasenbereichsränder sowie Blasenbereichsquerränder auf.

Vorzugsweise erstreckt sich der erste Längsabschnitt über eine Länge L1 ausgehend von einem ersten Manschettenquerrand bis zu einem endabschnittnahen Blasenbereichsquerrand. Der Endabschnitt bezeichnet denjenigen Abschnitt, der sich in Längsrichtung ausgehend vom endabschnittnahen Blasenbereichsquerrand, also an den ersten Längsabschnitt anschließend, bis zu einem zweiten Manschettenquerrand erstreckt. Vorzugsweise beträgt die Länge L1 des ersten Längsabschnitts, also dessen Erstreckung in der Längsrichtung 10 cm bis 40 cm, insbesondere 20 cm bis 30 cm, weiter insbesondere 22 cm bis 25 cm.

Der sich an den ersten Längsabschnitt in der Längsrichtung anschließende Endabschnitt weist vorzugsweise keine expandierbare Blase auf. Die Länge des Endabschnitts beträgt vorzugsweise 10 cm bis 35 cm, insbesondere 12 cm bis 20 cm.

In einer bevorzugten Ausführungsform umfasst die Wickelmanschette im ersten Längsabschnitt mindestens ein Schalenelement. Das mindestens eine Schalenelement erstreckt sich vorzugsweise über 50 % - 100 % der Fläche des ersten Längsabschnitts. Das eine oder die mehreren Schalenelemente sind bevorzugt aus einem biegesteifen Kunststoff gefertigt und versteifen somit den ersten Längsabschnitt. Der Begriff "biegesteif" bezeichnet im Rahmen dieser Anmeldung einen Körper, der zwar von einem Benutzer leicht biegbar ist, aber dem Biegevorgang einen Widerstand entgegensetzt. Weiterhin kehrt ein solcher biegesteifer Körper nach dem Ende einer Verbiegung, also ohne auf den Körper einwirkende äußere Kräfte, wieder zu seiner vorgeformten Gestalt zurück. Vorzugsweise zwingen das eine oder die mehreren Schalenelemente der Manschette eine vorgeformte Rundung auf. Solchenfalls kann die Manschette durch die vorgeformte Rundung aus im Wesentlichem dem ersten Längsabschnitt einen Körper in Form eines Zylindermantels ausbilden, wobei sich im Inneren des Zylindermantels ein Hohlraum befindet. Dieser Hohlraum kann den Oberarm des Benutzers aufnehmen.

In einer ganz besonders bevorzugten Variante erstreckt sich das mindestens eine Schalenelement über 50 % - 100 %, insbesondere 65 % bis 98 % und ganz besonders bevorzugt 80 % bis 95 % der Fläche des ersten Längsabschnitts. Solchenfalls ist ein Großteil der Fläche des ersten Längsabschnitts durch das eine oder die mehreren Schalenelemente versteift. Insbesondere ist es bevorzugt, wenn das eine oder die mehreren Schalenelemente im flachgelegten Zustand der Manschette die Blase vollständig überfangen.

Der untere Blasenbereichsrand und die Aussparung weisen in Querrichtung, welche senkrecht zu der Längsrichtung verläuft, einen ersten Abstand von 1,5 bis 5,5 cm, vorzugsweise von 2,5 bis 4,0 cm und insbesondere von höchsten 3,0 cm, voneinander auf. Dies ist besonders vorteilhaft, weil es im angelegten Zustand der Manschette einen geringen Abstand zwischen Blase und Ellenbeuge des Benutzers gewährleistet und so ein Verrutschen der Manschette in die Ellenbeuge hinein verhindert. Würde die Blase der angelegten Manschette in dem Bereich der Ellenbeuge mit einem Fluid befüllt werden, so würde dies zu einer ungenauen Messung des Blutdrucks führen. Diese Ausführungsform erlaubt durch den ersten Abstand eine vorteilhafte Positionierung der Manschette in Querrichtung. Der Begriff "Abstand", bezogen auf die Querrichtung, bezeichnet in dem Rahmen dieser Erfindung dabei stets die Entfernung zwischen zwei in Längsrichtung gezogenen, parallelen Hilfslinien durch die beiden Punkte, deren Abstand gemessen werden soll.

In einer bevorzugten Ausführungsform weist die Manschette eine rechteckige oder trapezförmige Kontur der Aussparung auf.

In einer besonders bevorzugten Ausführungsform weist die Manschette eine im Wesentlichen bogen- oder kurvenförmige Kontur der Aussparung auf. Weitere Konturen der Aussparung, insbesondere Kombinationen aus kurvenförmigen und geradlinigen Konturabschnitten sind ebenfalls denkbar und vorteilhaft.

Es umfasst die Positionierhilfe einen ersten Flügel und einen zweiten Flügel, wobei die Aussparung in Längsrichtung beidseitig durch die Flügel begrenzt ist. Dies ist für den Benutzer von besonderem Vorteil, da die Flügel im angelegten Zustand der Manschette, in dem die Aussparung an der Ellenbeuge des Benutzers anliegt, über den Außenseiten des Ellenbogengelenks des Benutzers zu liegen kommen und die korrekte Position der Manschette zusätzlich stabilisieren. Die Flügel werden dadurch gebildet, dass der erste Längsabschnitt im Bereich der Positionierhilfe sich vorzugsweise weiter in Querrichtung nach außen erstreckt als ein Bereich außerhalb der Positionierhilfe, so dass die Manschette im Bereich der Positionierhilfe eine höhere Breite aufweist als ein der Positionierhilfe benachbarter Bereich der Manschette, insbesondere als der Endabschnitt. Die Breite B1 des ersten Längsabschnitts im Bereich der Positionierhilfe beträgt insbesondere bevorzugt 11 cm bis 24 cm, weiter insbesondere 14 cm bis 20 cm.

Besonders bevorzugt ist es hierbei, wenn die Positionierhilfe in Längsrichtung eine Länge von 10 cm bis 25 cm, noch mehr bevorzugt von 14 cm bis 18 cm aufweist.

Ebenfalls besonders vorteilhaft ist es, wenn sich die Aussparung der Positionierhilfe in Querrichtung vom distalen Ende der Flügel bis zur in Querrichtung weitesten Erstreckung der Aussparung nach innen über eine Breite von wenigstens 2 cm und höchstens 7 cm, noch bevorzugter von wenigstens 3 cm und höchstens 5 cm erstreckt.

In einer anderen bevorzugten Weiterbildung der Erfindung weist die Positionierhilfe, vorzugsweise zumindest der erste und/oder der zweite Flügel, ein Versteifungselement auf oder wird davon gebildet, wobei das Versteifungselement die Positionierhilfe, insbesondere die Form der Flügel gegen Verknicken und Verbiegen stabilisiert. Dem Benutzer wird auf diese Weise beim Anlegen die korrekte Positionierung der Manschette erleichtert.

Das Versteifungselement ist dabei bevorzugt auf der Innenseite oder der Außenseite oder zwischen der die Innenseite und der die Außenseite der Positionierhilfe bildenden Materiallagen angeordnet. Des Weiteren ist es von Vorteil, wenn das Versteifungselement mindestens ein Metall oder mindestens einen Kunststoff beinhaltet oder ein mehrschichtiges Laminat umfasst. Vorteilhaft weist das Versteifungselement eine höhere Steifheit auf, als die die Positionierhilfe im Übrigen bildenden, insbesondere die die Innenseite und die Außenseite bildenden Materiallagen.

Das Versteifungselement weist in Querrichtung vorzugsweise einen zweiten Abstand von höchstens 6,0 cm vorzugsweise von höchsten 5,0 cm, insbesondere zwischen 0,25 cm und 4,0 cm, weiter insbesondere zwischen 0,8 cm und 1,5 cm von dem unteren Blasenbereichsrand auf.

In einer ganz besonders bevorzugten Ausführungsform kann das Versteifungselement durch eine Weitererstreckung des Schalenelements bis in den Bereich der Positionierhilfe hinein gebildet werden.

In einer Weiterbildung einer leicht anzulegenden Manschette umfasst diese in Längsrichtung zumindest in einem ersten oberen Randbereich einen Orientierungsbereich, der sich insbesondere durch Farbe, Material oder Steifigkeit von einem in Querrichtung an den Orientierungsbereich angrenzenden Bereich der Manschette unterscheidet. Dieser Orientierungsbereich zeigt dem Benutzer an, dass jener obere Randbereich beim Anlegen der Manschette in Richtung des Schultergelenks des Benutzers angeordnet werden soll. Es wird vermieden, dass die Manschette verkehrt herum angelegt wird und so durch eine unvorteilhafte Positionierung der Blase auf dem Bizeps (bzw. der Arteria brachialis) des Benutzers ungenaue Messwerte für den Blutdruck ermittelt werden.

In einer anderen bevorzugten Weiterbildung einer leicht anzulegenden Manschette umfasst diese daher eine zweite Anlegehilfe, die an einem Randbereich des Endabschnitts angeordnet ist. Die zweite Anlegehilfe weist ein zweites Anfassmittel auf, das sich in Farbe, Material oder Steifigkeit unterscheidet von einem Teilbereich des Endabschnitts, welches sich in Längsrichtung an das zweite Anfassmittel anschließt. Dieses zweite Anfassmittel ist daher für den Benutzer leicht zu erkennen und ermöglicht es, die Manschette im angelegten Zustand an dem Endabschnitt zu greifen und durch Zug zusammenzuziehen, vorzugsweise bis der innere Umfang der Manschette dem Umfang des Oberarms des Benutzers entspricht.

In einer weiteren bevorzugten Ausführungsform umfasst die Manschette einen Druckschlauch, an dem vorzugsweise ein insbesondere mindestens zweifarbiger Informationsträger angebracht ist. Dieser Informationsträger ist Träger von Informationen vermittelnden Kennzeichnungen, welche insbesondere auf den Informationsträger aufgedruckt sein können, insbesondere in Form von Piktogrammen oder ähnlichen Druckbildern. Die Informationen vermittelnden Kennzeichnungen veranschaulichen vorzugsweise das korrekte Anlegen der Manschette durch den Benutzer, vorzugsweise mit Bezug zu den beschriebenen Positionier-, Anlege- und Orientierungshilfen.

Derartige insbesondere zwei- oder mehrfarbige Informationsträger sind nach einem weiteren Erfindungsgedanken auch auf der Außenseite der Manschette angeordnet, insbesondere auf dem in angelegten Zustand auf dem Bizeps und Trizeps des Benutzers aufliegenden und damit gut sichtbaren ersten Längsabschnitt.

In einer weiteren besonders bevorzugten Ausführungsform umfasst die Manschette zumindest an dem ersten Längsabschnitt einen sich in der Längsrichtung erstreckenden Ablesebereich mit Ablesemarkierungen. Die Ablesemarkierungen können dabei insbesondere Linien, Punkte, Symbole, Farbmuster, Zahlen, Buchstabenfolgen oder Kombinationen aus den genannten Markierungen enthalten. Außerdem ist es von Vorteil, wenn die Ablesemarkierungen dabei bevorzugt auf der Außenseite, insbesondere an einem ersten oberen Randbereich angeordnet sind. Zieht nun der Benutzer beim Anlegen der Manschette an dem Endabschnitt, um den inneren Umfang der Manschette an den Umfang des Oberarms des Benutzers anzupassen, und schließt die Manschette dann durch Auflegen eines Haftbereichs des Endabschnitts auf einen Haftbereich des ersten Längsabschnitts, so zeigt die Position des Endabschnitts im Ablesebereich an, wie weit die Manschette zugezogen wurde. Dies ermöglicht es einem Benutzer, die individuell ermittelte Ablesemarkierung, die die Position des Endabschnitts passgenau anzeigt, zu dokumentieren oder zu erinnern. Bei der nächsten Messung des Blutdrucks dienen die Ablesemarkierungen dann dazu, die Manschette erneut auf denselben Umfang einzustellen. Auf diese Weise wird die Genauigkeit und Reproduzierbarkeit von Blutdruckmessungen, insbesondere durch nicht geschulte Personen, verbessert.

Besonders vorteilhaft ist es, wenn der Ablesebereich den Orientierungsbereich bildet oder mit diesem zusammenfällt. Die Ablesemarkierungen können dann in einer weiteren bevorzugten Ausführungsform der Erfindung Zahlen sein, die auf einem andersfarbigen Streifen an dem ersten oberen Rand des ersten Längsabschnitts angeordnet sind.

In einer Weiterbildung der Erfindung ist die Positionierhilfe an einer von der Manschette ablösbaren und wieder festlegbaren Positioniervorrichtung angeordnet. Die Positioniervorrichtung umfasst vorzugsweise ein Material mit zu dem im ersten Längsabschnitt verwendeten Schalenelement vergleichbarer Steifigkeit. Insbesondere umfasst die Positioniervorrichtung ein flaches Kunststoffmaterial. Das Kunststoffmaterial ist vorzugsweise ein biegesteifes, vorkonturiertes Kunststoffmaterial.

Eine mit der Positioniervorrichtung abnehmbare Positionierhilfe gestattet es, die Manschette bei Benutzern anzuwenden, welche die Positionierhilfe nicht einsetzen möchten. Auch zum Zwecke der Verpackung und Aufbewahrung ist es vorteilhaft, die Positionierhilfe von der Manschette trennen und wiederholt anbringen zu können.

Die Positioniervorrichtung weist hierzu vorzugsweise erste Befestigungsmittel auf, vermittels derer die Positioniervorrichtung auf der Manschette festlegbar ist. Vorzugsweise umfassen die ersten Befestigungsmittel an einem unteren Längsrand der Positioniervorrichtung angeordnete und sich in Querrichtung über diesen gleichsam halbinselartig hinaus erstreckende Vorsprünge, insbesondere zwei oder mehreren Vorsprünge.

Die Wickelmanschette weist solchenfalls vorzugsweise Taschen auf, wobei die Taschen auf ihrer dem ersten unteren Randbereich der Manschette zugewandten unteren Seite offen sind. Die Taschen können auf die Außenseite der Manschette aufgesetzt sein; denkbar und vorteilhaft wäre auch, die Taschen sandwichartig zwischen die beiden Materiallagen anzuordnen, welche die Innenseite und die Außenseite der Manschette bilden. Die Verwendung von die Vorsprünge aufnehmenden Laschen anstelle von Taschen wäre ebenfalls denkbar und vorteilhaft.

Die erste Befestigungsmittel bildenden Vorsprünge sind dann zur Fixierung der Positioniervorrichtung an der Manschette in die dafür vorgesehenen Taschen einschiebbar. Vorteilhafterweise umfassen die Vorsprünge und die Taschen miteinander kooperierende Rastmittel, wie Einrastnasen und korrespondierende Haltemittel, so dass die Vorsprünge in den Taschen in einer vorgegebenen Stellung gehalten sind.

Denkbar und vorteilhaft wäre auch, das lösbare Festlegen der Positioniervorrichtung an der Manschette alternativ zu den ersten Befestigungsmitteln oder in Ergänzung dazu durch auf der Innenseite der Positioniervorrichtung vorzusehende Haftbereiche, insbesondere Klettbereiche, vorzugsweise in Form von Kletthaken, zu ermöglichen, welche mit Haftbereichen, insbesondere Klettbereichen, vorzugsweise einem Flauschmaterial, der Außenseite des ersten Längsabschnitts in Eingriff bringbar sind.

In einer weiteren besonders bevorzugten Ausführungsform umfasst die Innenseite des ersten Längsabschnitts ein Haftmaterial oder wird davon gebildet, wobei dieses Haftmaterial der Innenseite des ersten Längsabschnitts auf der Haut eines Benutzers eine höhere Haftung als eine Außenseite des ersten Längsabschnitts verleiht. Diese Ausführungsform ist von Vorteil, weil sie einem Verrutschen der Manschette in Längsrichtung auf dem Oberarm des Benutzers entgegen wirkt.

Vorzugsweise umfasst der erste obere Randbereich des ersten Längsabschnitts der Manschette zum Anlegen an den Oberarm eines Menschen eine erste Anlegehilfe, die ein erstes Anfassmittel aufweist.

Vorzugsweise ist das erste Anfassmittel insbesondere durch seine Farbe, Material oder Steifigkeit von einem ersten unteren Randbereich des ersten Längsabschnitts unterscheidbar und dadurch für den Benutzer leicht zu identifizieren.

Das erste Anfassmittel ist vorzugsweise in Form einer Lasche, eines Griffs, eines Hakens, eines Henkels oder eines Bügels ausgebildet. Das erste Anfassmittel ermöglicht es dem Benutzer, beim Anlegen der Manschette die Manschette an den Oberarm eines Menschen nach oben, also in Richtung des Schultergelenks des Benutzers, zu ziehen, um eine passgenauere Positionierung in Querrichtung zu gewährleisten.

In einer Weiterbildung umfasst das erste Anfassmittel einen Greifbereich und einen Markierungsbereich, wobei der Markierungsbereich eine vom Greifbereich durch Farbe, Material oder Steifigkeit, insbesondere visuell unterscheidbare Greifmarkierung aufweist, an der der Benutzer das erste Anfassmittel zwischen Zeigefinger und Daumen greifen kann.

### Figurenteil

Nachstehend werden Ausführungsformen erfindungsgemäßer Wickelmanschetten mit Positionierhilfe anhand von Zeichnungen von flachgelegten Manschetten näher erläutert. Alle im Zusammenhang mit der vorliegenden Erfindung vor- und nachstehend beschriebenen Abmessungen wie Abstände, Längen oder Breiten werden an der flachgelegten Manschette vermessen, wobei die Blase noch unbefüllt, also noch nicht expandiert ist. Weiterhin sind Elemente wie das Schalenelement und die Positionierhilfe flachgedrückt, um eine vollständig flachgelegte Manschette zu gewährleisten. Die Erfindung soll jedoch nicht auf die in den Zeichnungen oder in der Beschreibung der Zeichnung dargestellten Ausgestaltungen reduziert verstanden werden. Es zeigen:
**Fig. 1a und 1b****)** Draufsichten auf erfindungsgemäße flachgelegte Wickelmanschetten für das Messen des Blutdrucks.
**Fig. 1c****)** Querschnitt, schematisch, durch die zylindermantelartig vorgeformte, nicht flachgelegte Wickelmanschette aus 1a entlang der Achse D-D.
**Fig. 2a****)** Draufsicht auf eine erfindungsgemäße Wickelmanschette mit Darstellung weiterer Details der Manschette.
**Fig. 2b****)** Querschnitt, schematisch, durch die Wickelmanschette entlang der Achse A-A aus Figur 2a.
**Fig. 3a****-d)** Schematische Darstellung verschiedener Konturen von Positionierhilfen erfindungsgemäßer Manschetten.
**Fig. 4a****)** Detailansicht einer erfindungsgemäßen Manschette mit einem Orientierungs- und einem Ablesebereich.
**Fig. 4b****-d)** Detailansichten von ersten Anlegehilfen.
**Fig. 5****)** Schematische Darstellung einer Wickelmanschette im angelegten Zustand auf dem Oberarm eines Benutzers.
**Fig. 6a****-c)** Draufsicht auf eine Positioniervorrichtung, und je eine Teildraufsicht auf eine Wickelmanschette vor und nach dem Festlegen der Positioniervorrichtung an der Manschette

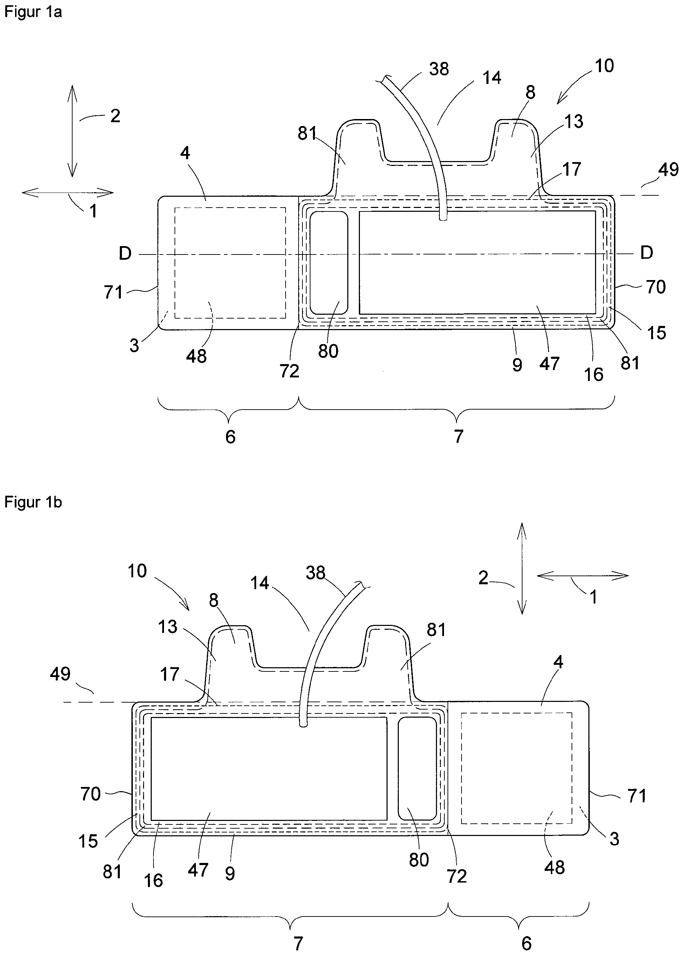

Figur 1a zeigt eine erfindungsgemäße Wickelmanschette 10 für das Messen des Blutdrucks mit einer Längsrichtung 1 und einer Querrichtung 2 und mit einer Innenseite 3 und einer Außenseite 4. Die Wickelmanschette 10 umfasst einen ersten Längsabschnitt 7 und einen Endabschnitt 6, der sich in Längsrichtung 1 an den ersten Längsabschnitt 7 anschließt. Der erste Längsabschnitt 7 weist ein Schalenelement 81 und einen ersten unteren Randbereich 8 sowie einen ersten oberen Randbereich 9 auf. Die Wickelmanschette 10 ist im flachgelegten Zustand dargestellt, wobei der erste Längsabschnitt 7 gegen den Widerstand des Schalenelements 81 flachgedrückt ist. Der erste Längsabschnitt 7 umfasst an einem ersten unteren Randbereich 8 eine Positionierhilfe 13, wobei die Positionierhilfe 13 eine Aussparung 14 aufweist. Weiterhin umfasst die Wickelmanschette 10 einen Blasenbereich 15 mit einem unteren Blasenbereichsrand 17, wobei der Blasenbereich 15 eine Blase 16 aufweist. Die Blase 16 ist fluidleitend mit einem Druckschlauch 38 verbunden.

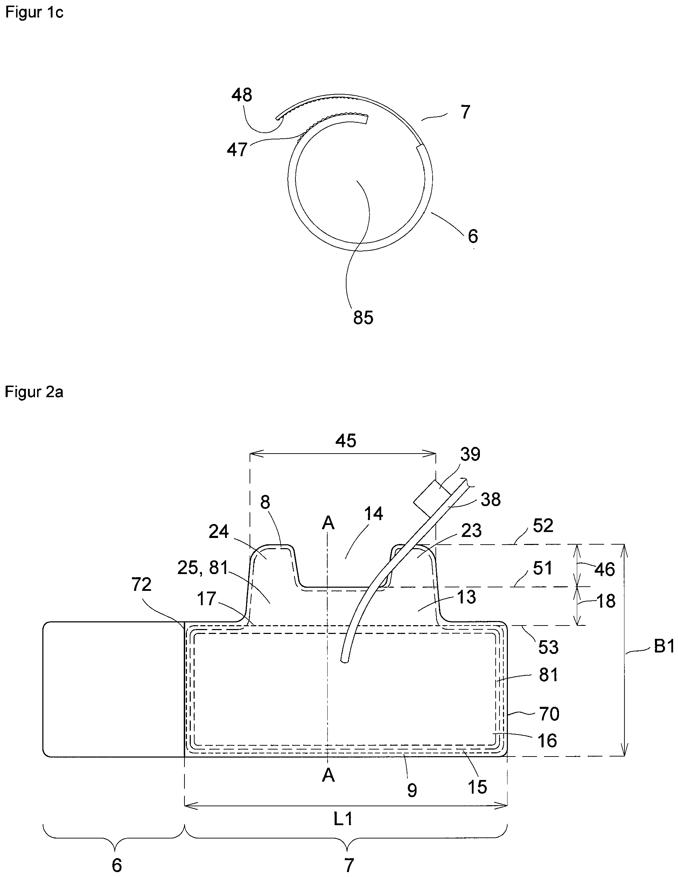

Um die Manschette 10 anzulegen, muss ein Benutzer zunächst den ersten Längsabschnitt 7 der Manschette 10 auf den Oberarm auflegen. Die Manschette 10 weist im ersten Längsabschnitt 7 ein Schalenelement 81 auf, welches der Manschette 10 eine vorgeformte Rundung aufzwingt (Figur 1c). Das Schalenelement 81 ist vorzugsweise aus einem biegesteifen Kunststoff gefertigt. Durch die vorgeformte Rundung bildet die Manschette 10 aus im Wesentlichem dem ersten Längsabschnitt 7 einen Körper in Form eines Zylindermantels aus, wobei sich im Inneren des Zylindermantels ein Hohlraum 85 befindet (Figur 1c). Dieser Hohlraum kann den Oberarm des Benutzers aufnehmen. Der Innenumfang des Zylindermantels bzw. der angelegten Manschette wird dann durch Zug an dem Endabschnitt 6 auf den Umfang des Oberarms des Benutzers angepasst. Der Endabschnitt 6 kann dann wiederablösbar auf dem ersten Längsabschnitt festgelegt werden, wobei der Endabschnitt 6 auf der Innenseite 3 einen ersten Haftbereich 48 und der erste Längsabschnitt 7 auf der Außenseite 4 einen zweiten Haftbereich 47 aufweisen. Diese ersten und zweiten Haftbereiche 47,48 sind in der dargestellten bevorzugten Ausführung der Manschette 10 durch erste und zweite miteinander in Eingriff bringbare Klettbereiche gebildet. Der erste Klettbereich ist vorzugsweise in Form einer schlaufenbildenden Zone 47 gebildet. Der zweite Klettbereich ist solchenfalls in Form einer Kletthaken aufweisenden Zone 48 gebildet. Im angelegten Zustand der Manschette 10 umfasst der erste Längsabschnitt 7 im Wesentlichen jenen Bereich der Manschette 10, der bei korrekter Positionierung der Manschette 10 im angelegten Zustand auf dem Bizeps und dem Trizeps des Benutzers zu liegen kommt. Vorzugsweise erstreckt sich der erste Längsabschnitt 7 wie in Figur 2a dargestellt über eine Länge L1 ausgehend von einem ersten Manschettenquerrand 70 bis zu einem endabschnittnahen Blasenbereichsquerrand 72. Der Endabschnitt 6 bezeichnet denjenigen Abschnitt, der sich in Längsrichtung 1 ausgehend vom endabschnittnahen Blasenbereichsquerrand 72, also an den ersten Längsabschnitt 7 anschließend, bis zu dem zweiten Manschettenquerrand 71 erstreckt. Die sich in der Längsrichtung 1 aneinander anschließenden Abschnitte (erster Längsabschnitt 7, Endabschnitt 6) sind vorzugsweise integral aus einem einzigen, vorzugsweise mehrlagigen Materialabschnitt gebildet. Es wäre aber auch denkbar, dass die Abschnitte aus unterschiedlichen Materialabschnitten gebildet sind, die zur Bildung der Wickelmanschette aneinander gefügt sind. Weiterhin umfasst der erste Längsabschnitt 7 in der Nähe des endabschnittnahen Blasenbereichsquerrands 72 einen Informationsträger 80, der durch die Außenseite 4 der Manschette 10 gebildet wird oder auf der Außenseite 4 angeordnet ist. Der Informationsträger 80 ist Träger von Informationen vermittelnden Kennzeichnungen, welche insbesondere auf den Informationsträger aufgedruckt sein können, insbesondere in Form von Piktogrammen oder ähnlichen Druckbildern. Die Informationen vermittelnden Kennzeichnungen veranschaulichen vorzugsweise das korrekte Anlegen der Manschette durch den Benutzer, vorzugsweise mit Bezug zu den beschriebenen Positionier-, Anlege- und Orientierungshilfen. Der Informationsträger 80 ist vorzugsweise zwei- oder mehrfarbig ausgestaltet. Die Außenseite 4 des ersten Längsabschnitts 7 in der Nähe des endabschnittnahen Blasenbereichsquerrands 72 kommt im angelegten Zustand der Manschette 10 auf dem Bizeps und Trizeps des Benutzers zu liegen. Die Anordnung des Informationsträgers 80 an vorgenannter Stelle gewährleistet somit insbesondere im angelegten Zustand der Manschette 10 ein gute Sicht- und Ablesbarkeit des Informationsträgers 80.

Der erste Längsabschnitt 7 umfasst an einem ersten unteren Randbereich 8 eine Positionierhilfe 13, wobei die Positionierhilfe 13 eine Aussparung 14 aufweist. Das den ersten Längsabschnitt bildende Material ist also an dem unteren Randbereich 8, welcher im angelegten Zustand dem Unterarm zugewandt ist, zur Bildung einer Positionierhilfe ausgespart. Durch die Aussparung 14 kann die Manschette 10 so am Innenbereich der Ellenbeuge des Benutzers angelegt werden, dass die Gefahr eines Verrutschens der Manschette 10 auf dem Arm in Längsrichtung 1 (also im angelegten Zustand in Umfangsrichtung) vermindert wird. Auf diese Weise ist die Manschette, insbesondere die Blase 16, in vorteilhafter Weise so positioniert, dass eine genaue und verlässlich reproduzierbare Messung des Blutdrucks gewährleistet ist.

Die Positionierhilfe 13 ist in dieser Variante durch eine Weitererstreckung des ersten Längsabschnitts 7 in einem dem ersten Manschettenquerrand 70 benachbarten Bereich in Querrichtung 2 über eine erste gedachte Linie 49 hinaus gebildet, wobei die gedachte Linie 49 im Wesentlichen entlang der Kontur des unteren Randes des Endabschnitts 6 verläuft. Der erste Längsabschnitt 7 erstreckt sich damit im Bereich der Positionierhilfe 13 weiter in Querrichtung 2 nach außen, also nach unten (im angelegten Zustand in Richtung auf die Ellenbeuge), als ein Bereich außerhalb der Positionierhilfe 13, so dass der Manschette 10 im Bereich der Positionierhilfe 13 eine höhere Breite B1 (Figur 2a) aufweist als ein der Positionierhilfe 13 in Längsrichtung 1 benachbarter Bereich des ersten Längsabschnitts 7 der Manschette 10, und/oder als der Endabschnitt 6.

Die Breite B1 (Figur 2a) des ersten Längsabschnitts 7 im Bereich der Positionierhilfe 13 beträgt bevorzugt 11 cm bis 24 cm, insbesondere 14 cm bis 20 cm.

Die Aussparung 14 hat im dargestellten Fall eine im Wesentlichen bogenförmige Kontur.

Figur 1a zeigt eine Wickelmanschette 10 mit einem Endabschnitt 6 für das Schließen der Manschette 10 auf der rechten Seite, was bei dem hier beschriebenen Manschettentyp der Schalenmanschette besonders für ein Anlegen der Manschette auf dem linken Arm des Benutzers geeignet ist. Figur 1b zeigt eine Wickelmanschette 11 mit einem Endabschnitt 6 für das Schließen der Manschette 10 auf der linken Seite, was bei dem hier beschriebenen Manschettentyp der Schalenmanschette besonders für ein Anlegen der Manschette auf dem rechten Arm des Benutzers geeignet ist. Im Übrigen sind die in Figuren 1a und 1b dargestellten Manschetten baugleich. Gleiche Bezugszeichen kennzeichnen in Figur 1b dieselben im Zusammenhang mit Figur 1a beschriebenen Komponenten der Manschette.

Im Folgenden werden Ausführungsformen nur der rechtsseitigen Manschette 10 dargestellt und beschrieben. Sämtliche im Zusammenhang mit der rechtsseitigen Manschette 10 beschriebenen Merkmale und Varianten sind auch im Zusammenhang mit der Ausführung linksseitiger Manschetten 11 denkbar und vorteilhaft.

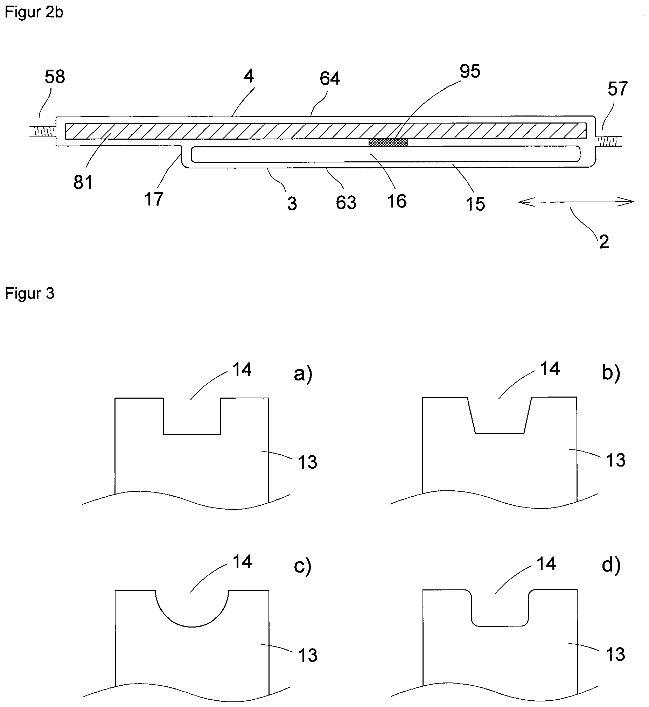

Die Manschette 10 umfasst außerdem eine in einen Blasenbereich 15 aufgenommene Blase 16 sowie einen Druckschlauch 38, welche nachfolgend anhand der Figuren 2a und 2b näher beschrieben werden.

In Figur 2a ist erkennbar und dargestellt, dass die Positionierhilfe 13 einen ersten Flügel 23 und einen zweiten Flügel 24 umfasst, wobei die Aussparung 14 in Längsrichtung 1 beidseitig durch die Flügel 23, 24 begrenzt ist. In einem zentralen Bereich des ersten Längsabschnitts 7 vorzugsweise in der Flucht der Aussparung 14 ist ein Druckschlauch 38 angeordnet, an dem ein vorzugsweise mindestens zweifarbiger Informationsträger 39 in Form eines kleinen Fähnchens angebracht ist. Der Druckschlauch 38 ist mit der Blase 16 fluidleitend verbunden, so dass vermittels eines nicht dargestellten Gerätes, ein Fluid über den Druckschlauch 38 der Blase 16 zugeführt werden kann, wodurch die Blase expandierbar ist. Das nicht dargestellte Gerät umfasst vorzugsweise eine Pumpvorrichtung, welche das Fluid in den Druckschlauch zwingt. Das Gerät umfasst in ans sich bekannter Weise vorzugsweise außerdem Anzeigemittel wie ein Display, an dem ein ermittelter Blutdruck angezeigt werden kann und vorzugsweise elektronische Steuermittel, welche den Messvorgang der Blutdruckmessung steuern.

Das Versteifungselement 25 ist im vorliegenden Fall durch eine Weitererstreckung des Schalenelements 81 in den Bereich der Positionierhilfe 13 hinein gebildet. Es ist als eine dünne, entsprechend konturierte Hartkunststoffplatte ausgeführt. Das Versteifungselement 25 bzw. das Schalenelement 81 versteift die ansonsten flexible innere 63 und äußere Materiallage 64. Hierdurch wird die Positionierhilfe 13, insbesondere vorteilhaft die Form der Flügel 23, 24 gegen Verknicken und Verbiegen stabilisiert. Dem Benutzer wird auf diese Weise beim Anlegen die korrekte Positionierung der Manschette 10 erleichtert. Das Schalenelement 81 ist im ersten Längsabschnitt 7 zwischen die beiden Materiallagen 63, 64 eingelegt und wird durch die Außenränder bzw. Außennähte des ersten Längsabschnitts 7 in Position gehalten (Figur 2b). So können die beiden Materiallagen 63,64 beispielsweise am ersten oberen Randbereich 9 der Manschette 10 mit einer ersten Naht 57 vernäht oder thermoverschweißt sein. Im ersten unteren Randbereich 8 kann eine zweite Naht 58 vorgesehen sein. Vorzugsweise wird die Blase 16 in dem Blasenbereich 15 gegen Verrutschen geschützt, indem ein kleines zweiseitig klebendes Klebeband 95 die Blase an dem Schalenelement 81 fixiert.

Besonders vorteilhaft ist es, wenn die Positionierhilfe 13 für den Fall, dass die Positionierhilfe sich weiter in Querrichtung nach außen, also nach unten (im angelegten Zustand in Richtung auf die Ellenbeuge), als ein Bereich außerhalb der Positionierhilfe erstreckt, so dass die Manschette im Bereich der Positionierhilfe eine höhere Breite B1 aufweist als ein der Positionierhilfe 13 in Längsrichtung 1 benachbarter Bereich des ersten Längsabschnitts 7 der Manschette 10, in Längsrichtung 1 eine Länge 45 von 10 cm bis 25 cm, noch mehr bevorzugt von 14 cm bis 18 cm aufweist. Die Länge 45, also die maximale Erstreckung der Positionierhilfe in der Längsrichtung 1, wird dabei wie in Figur 2a verdeutlicht im Rahmen der vorliegenden Erfindung solchenfalls dort gemessen, wo die Aussparung 14 sich in Querrichtung 2 am weitesten nach innen in den ersten Längsabschnitt 7 hinein erstreckt.

Die Breite 46 der Aussparung 14 beträgt vorteilhaft wenigstens 2 cm und höchstens 7 cm, noch vorteilhafter wenigstens 3 cm und höchstens 5 cm. Die Breite 46 der Aussparung wird im Rahmen der vorliegenden Erfindung als der Abstand zwischen zwei weiteren gedachten, in der Längsrichtung verlaufenden Linien 51 und 52 verstanden. Hierbei verläuft eine gedachte Aussparungslinie 51 dort, wo die Aussparung 14 sich in Querrichtung 2 am weitesten nach innen in den ersten Längsabschnitt 7 hinein erstreckt. Eine gedachte Flügellinie 52 verläuft durch das distale Ende der Flügel 23, 24, also dort, wo die Flügel sich am weitesten nach außen erstrecken. Sollte sich einer der Flügel weiter nach außen erstrecken als der andere, so wird die gedachte Flügellinie an das distale Ende desjenigen Flügels angelegt, der sich weiter nach außen erstreckt.

Figur 2b zeigt überdies, dass die Manschette 10 zwei aneinander gefügte Materiallagen 63 und 64 umfasst, wobei die innere Materiallage 63 die Innenseite 3 und die äußere Materiallage 64 die Außenseite 4 der Manschette 10 bilden. Die inneren und äußeren Materiallagen 63,64 können textile Stoffe, Folien, Nonwoven oder andere flexible, anschmiegsame Materialien umfassen und ihrerseits vorteilhaft ein- oder mehrschichtig ausgebildet sein. Zwischen die innere und äußere Materiallage 63, 64 ist in einem Blasenbereich 15 eine Blase 16 aufgenommen.

Der Blasenbereich 15 ist im dargestellten Fall dadurch gebildet, dass entlang einer vorzugsweise in etwa rechteckförmigen Kontur die beiden Materiallagen 63, 64 durch eine oder mehrere Nähte (nicht dargestellt) oder andere Fügeverfahren wie insbesondere Thermoschweißen oder Verkleben unter Ausbildung der Blasenbereichsränder miteinander verbunden sind.

Die Aussparung 14 ist in Querrichtung 2 mit einem ersten Abstand 18 vom unteren Blasenbereichsrand 17 beabstandet, und zwar vorzugsweise um mindestens 1,5 cm, vorzugsweise um 1,5 bis 5,5 cm, weiter vorzugsweise um 2,5 bis 4,0 cm und insbesondere um höchsten 3,0 cm. Der erste Abstand 18 ist hierbei stets der Abstand (also die kürzeste Entfernung) zwischen der gedachten Aussparungslinie 51 und einer weiteren in der Längsrichtung 1 verlaufenden gedachten Blasenbereichslinie 53, welche dort verläuft, wo sich der untere Blasenbereichsrand 17 in Querrichtung 2 am weitesten nach außen, also in Richtung auf die Positionierhilfe 13 erstreckt.

Vorzugsweise beträgt die Länge L1 des ersten Längsabschnitts 7, also dessen maximale Erstreckung in der Längsrichtung 10 cm bis 40 cm, insbesondere 20 cm bis 30 cm, weiter insbesondere 22 cm bis 25 cm. Die Länge L1 des ersten Längsabschnitts wird solchenfalls ermittelt als der Abstand von einem ersten Manschettenquerrand 70 bis zu einem endabschnittnahen Blasenbereichsquerrand 72. Die Länge des verbleibenden Endabschnitts 6 beträgt vorzugsweise 10 cm bis 35 cm, insbesondere 24 cm bis 28 cm. Die Gesamtlänge der Manschette 10 resultiert mithin aus der Summe der Längen des ersten Längsabschnitts 7 und des Endabschnitts 6.

Figuren 3a-d veranschaulichen verschiedene bevorzugte Ausführungen der Positionierhilfe 13. Die Aussparung 14 weist vorzugsweise eine im Wesentlichen rechteckige (Figur 3a) oder trapezförmige (Figur 3b) Kontur auf. Auch eine im Wesentlichen bogen- (Figur 3c) oder kurvenförmige Kontur (Figur 3d) ist vorteilhaft. Unterschiedliche Varianten der Aussparung sind dadurch besonders vorteilhaft, dass abhängig von der Form und Größe der Ellenbeuge eines Benutzers eine Positionierhilfe 13 gewählt werden kann, die im angelegten Zustand der Manschette möglichst passgenau in der Ellenbeuge des Benutzers zu liegen kommt. Andere, nicht dargestellte Konturen der Aussparung, insbesondere Kombinationen aus kurvenförmigen und geradlinigen Konturabschnitten sind ebenfalls denkbar und vorteilhaft.

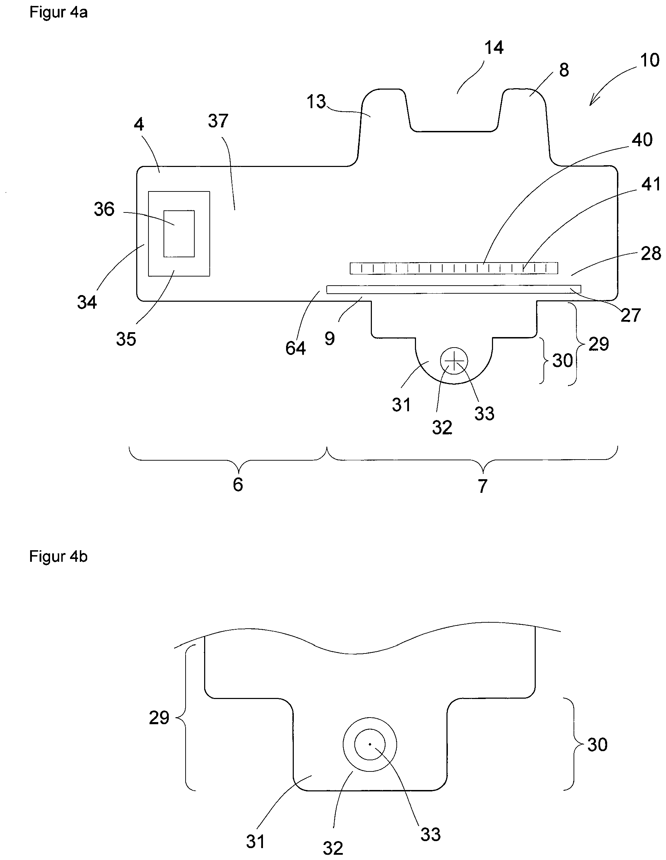

In Figur 4a ist eine weitere Variante der erfindungsgemäßen Wickelmanschette 10 dargestellt (in Figur 4a und den nachfolgenden Figuren 4b-4d sind jeweils nicht alle Komponenten der Manschette dargestellt, um die Figuren nicht zu überfrachten).

Die Manschette 10 umfasst an einem ersten unteren Randbereich 8 eines ersten Längsabschnitts 7 eine Positionierhilfe 13 mit einer Aussparung 14. Die Manschette 10 umfasst außerdem (nicht dargestellt) einen Blasenbereich mit einer expandierbaren Blase, Haftbereiche auf der Außenseite 4 des ersten Längsabschnitts 7 zum Fixieren der Manschette um den Oberarm eines Benutzers und gegebenenfalls eine oder mehrere der weiteren Komponenten, vorzugsweise wie vorstehend im Zusammenhang mit den Figuren 1-3 beschrieben.

Weiterhin weist die Manschette 10 zumindest an einem ersten oberen Randbereich 9 einen sich in der Längsrichtung 1 erstreckenden Orientierungsbereich 27 auf (Figur 4a), der sich durch Farbe, Material oder Steifigkeit von einem in Querrichtung 2 in Richtung der Positionierhilfe 13 an den Orientierungsbereich 27 angrenzenden Bereich 28 der Manschette unterscheidet. Bevorzugt handelt es sich bei dem Orientierungsbereich 27 um einen farblich abgesetzten Streifen, insbesondere einen roten oder orangefarbenen Streifen, der auf der Außenseite 4 der Manschette angebracht ist und entweder durch die Materiallage 64 der Außenseite 4 selbst gebildet wird oder auf dieser befestigt ist. Hierdurch wird es einem Benutzer der Manschette erleichtert, den ersten oberen Randbereich 9 zu erkennen und die Wickelmanschette richtig herum um den Oberarm anzulegen. Somit wird die Gefahr einer nicht korrekten Orientierung der Manschette verringert und die Genauigkeit einer Blutdruckmessung damit erhöht.

Weiterhin umfasst die dargestellte Wickelmanschette zumindest an dem ersten Längsabschnitt 7 einen sich in der Längsrichtung 1 erstreckenden Ablesebereich 40 mit Ablesemarkierungen 41 (Figur 4a). Die Ablesemarkierungen 41 werden vorzugsweise durch Linien, Punkte, Symbole, Farbmuster, Zahlen, Buchstabenfolgen oder Kombinationen aus den genannten Markierungen gebildet. Bevorzugt ist es, wenn die Ablesemarkierungen Zahlen aufweisen, die in Form einer Skala auf dem Ablesebereich 40 angeordnet sind und sich in ihrer Farbe vom Hintergrund des Ablesebereichs 40 abheben. Hierdurch kann der Benutzer der Manschette zum Messen des Blutdrucks den Umfang der angelegten Manschette ablesen und erinnern oder aufzeichnen, um bei der nächsten Messung des Blutdrucks wieder den gleichen Umfang der Manschette einstellen zu können. Auf diese Weise wird die Reproduzierbarkeit von Blutdruckmessungen, insbesondere durch nicht geschulte Benutzer, erhöht.

Besonders bevorzugt ist es außerdem, wenn der Ablesebereich 40 mit dem Orientierungsbereich 27 derart kombiniert ist, dass der Ablesebereich 40 Zahlen umfasst, die auf einem andersfarbigen Streifen in dem ersten oberen Randbereich 9 des ersten Längsabschnitts 7 angeordnet sind, so dass Ablese- und Orientierungsbereich zusammenfallen (nicht dargestellt in Figur 4a).

Des Weiteren umfasst der Endabschnitt 6 vorzugsweise wie dargestellt (Figur 4a) an einem Randbereich 34 des Endabschnitts 6 eine zweite Anlegehilfe 35, die ein zweites Anfassmittel 36 aufweist. Dieses unterscheidet sich in Farbe, Material oder Steifigkeit von einem Teilbereich 37 des Endabschnitts 6, welcher sich in Längsrichtung 1 an das zweite Anfassmittel 36 in Richtung auf den ersten Längsabschnitt 7 anschließt. Hierdurch wird es dem Benutzer erleichtert, so am zweiten Anfassmittel 36 des Endabschnitts 6 zu ziehen, dass der korrekte Umfang der Manschette auf dem Oberarm des Benutzers eingestellt wird.

Der erste obere Randbereich 9 umfasst außerdem vorzugsweise eine erste Anlegehilfe 29, die ein, insbesondere daran angefügtes, erstes Anfassmittel 30 aufweist oder daraus gebildet ist. Diese so konfigurierte erste Anlegehilfe 29 stellt dabei einen an sich unabhängigen Erfindungsgedanken dar. Die erste Anlegehilfe 29 kann somit vorzugsweise auch bei an sich üblichen Manschetten zum Messen des Blutdrucks vorgesehen sein, also insbesondere solchen Manschetten, die keine Positionierhilfe 13 mit Aussparung 14 aufweisen.

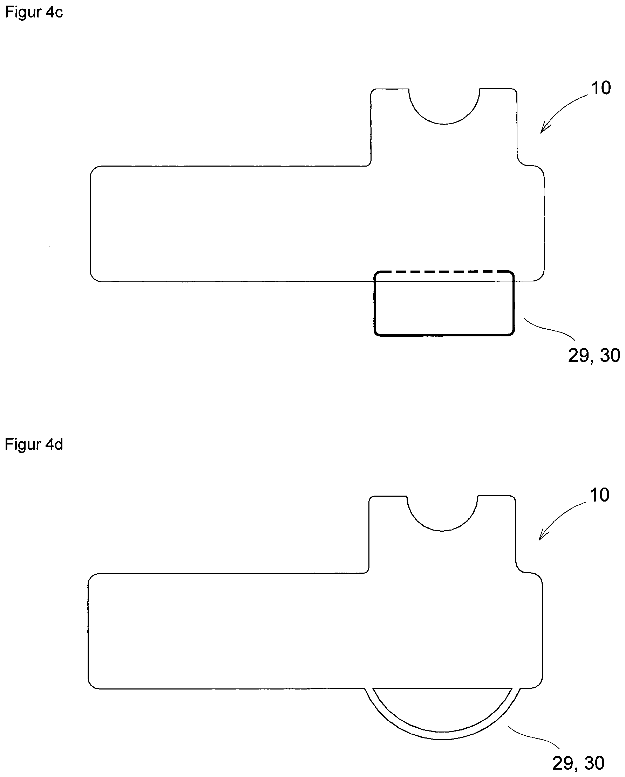

Das erste Anfassmittel 30 umfasst bevorzugt eine Lasche (Figuren 4a, 4b), einen Bügel (Figur 4c), einen Griff, einen Henkel (Figur 4d) oder einen Haken.

Ist das erste Anfassmittel 30 als ein Bügel oder ein Griff oder ein Haken ausgeführt, so umfasst dieser bevorzugt ein Metall oder einem Kunststoff mit hoher Biegesteifigkeit. Die Befestigung an dem ersten oberen Randbereich 9 erfolgt solchenfalls bevorzugt durch eine oder mehrere Taschen, die das erste Anfassmittel 30 mit der Wickelmanschette 10 verbinden.

Bei einer Ausführung des ersten Anfassmittels 30 als ein Henkel (Figur 4d) umfasst der Henkel bevorzugt ein flexibles Kunststoffmaterial oder Textil. Besonders bevorzugt ist es, wenn der Henkel durch aus dem gleichen Material wie die inneren und äußeren Materiallagen 63,64 besteht oder aus diesen Materiallagen gebildet wird.

Bei einer Ausführung des ersten Anfassmittels 30 als Lasche (Figur 4a,4b) ist diese bevorzugt so gestaltet, dass sie eine geringe Biegesteifigkeit aufweist. Dabei beinhaltet das erste Anfassmittel 30 vorzugsweise einen Greifbereich 31. Innerhalb des Greifbereichs 31 ist vorzugsweise ein Markierungsbereich 32 vorgesehen, wobei der Markierungsbereich 32 vorzugsweise eine vom Greifbereich 31 durch Farbe, Material oder Steifigkeit, insbesondere visuell unterscheidbare Greifmarkierung 33 aufweist. Hierdurch wird des dem Benutzer erleichtert, einen zum Anfassen besonders geeigneten Bereich zu erkennen.

In einer bevorzugten Ausführungsform ist diese Greifmarkierung 33 ein aufgedrucktes Symbol, wie beispielsweise ein Kreuz (Figur 4a), das eine andere Farbe als der Greifbereich 31 im Übrigen aufweist. In einer weiteren Ausführungsform ist diese Greifmarkierung 33 ein Kreis oder weist mehrere konzentrische Kreise (Figur 4b) mit einer anderen Farbe als der Greifbereich 31 auf. Hierdurch wird es dem Benutzer erleichtert, an einer besonders vorteilhaften Stelle der Manschette 10, nämlich der Greifmarkierung, anzufassen und die Manschette durch Ziehen in Richtung des Schultergelenks korrekt auf dem Oberarm zu positionieren. Der Benutzer kann hierbei die Greifmarkierung 33 zwischen Daumen und Zeigefinger angreifen, um Zug auszuüben.

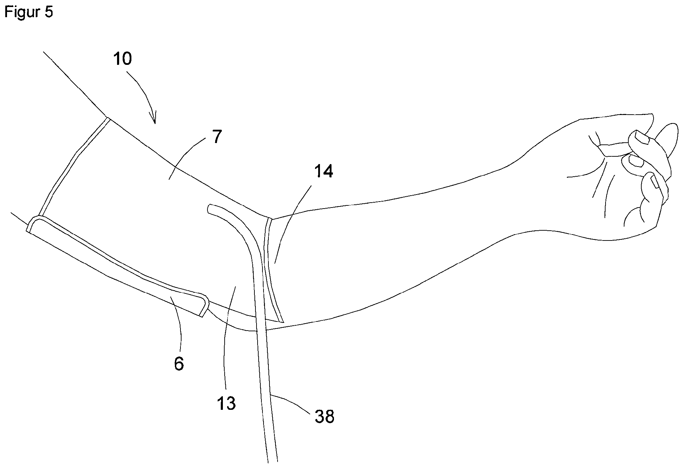

Figur 5 veranschaulicht den Sitz einer Manschette 10 für das Messen des Blutdrucks auf dem Oberarm eines Benutzers, also im angelegten Zustand der Manschette. Im angelegten Zustand bildet die Manschette einen zylindermantelartigen Hohlkörper aus, der den Oberarm des Benutzers aufnimmt.

Die Manschette ist solchenfalls durch wiederablösbares Festlegen des Endabschnitts 6 auf dem ersten Längsabschnitt 7 geschlossen. Ein Verrutschen der Manschette in Umfangsrichtung auf dem Oberarm des Benutzers wird vermindert oder sogar ganz verhindert, indem die Aussparung 14 der Positionierhilfe 13 in der Innenseite der Ellenbeuge des Benutzers anliegt. Dargestellt ist weiterhin ein Druckschlauch 38.

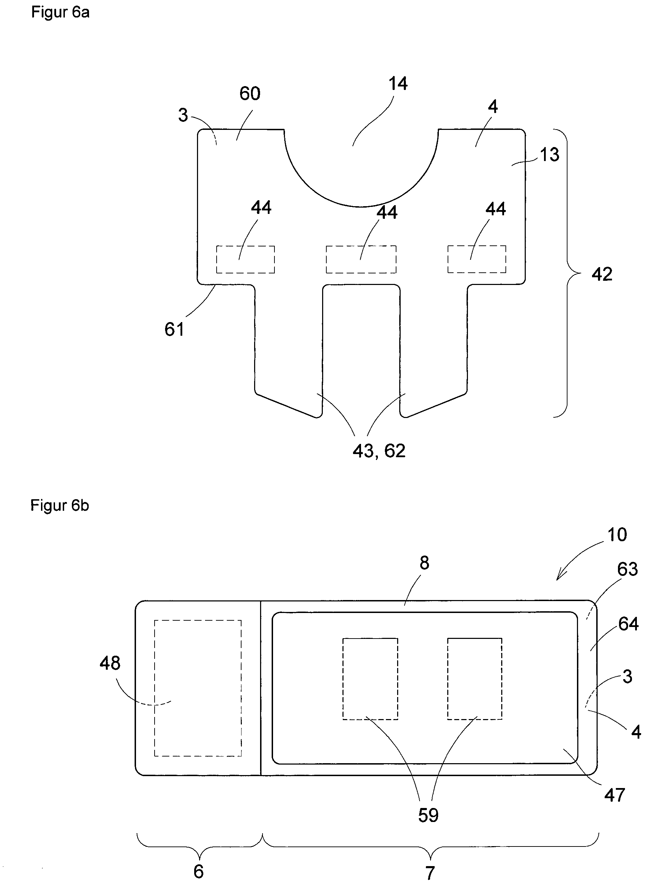

Figur 6a zeigt eine separate, die spätere Positionierhilfe 13 aufweisende Positioniervorrichtung 42 zum lösbaren Festlegen an einer Wickelmanschette. Hierzu weist die Positioniervorrichtung 42 an ihrem äußeren Längsrand 60 eine Positionierhilfe 13, umfassend eine Aussparung 14, auf. An einem inneren Längsrand 61 weist die Positioniervorrichtung 42 bevorzugt mindestens zwei Vorsprünge 62 auf, welche erste Befestigungsmittel 43 bilden.

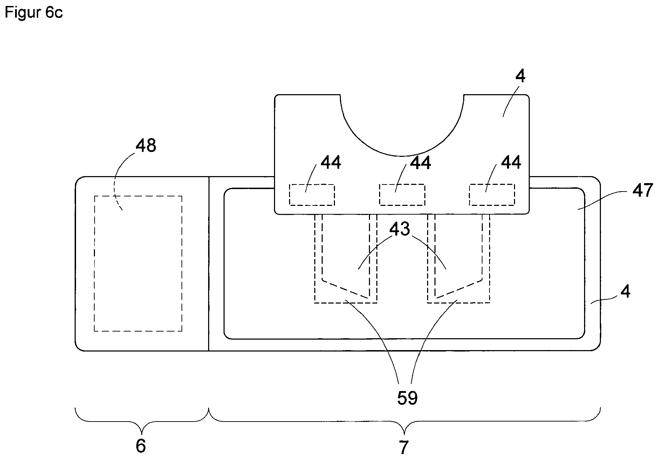

Die Wickelmanschette 10 (Figur 6b) weist solchenfalls in dem ersten Längsabschnitt 7 vorzugsweise Taschen 59 auf, wobei die Taschen 59 auf ihrer dem ersten unteren Randbereich 8 der Manschette zugewandten Seite offen sind. Die Taschen können auf die Außenseite 4 der Manschette 10 aufgesetzt, beispielsweise aufgenäht sein; denkbar und vorteilhaft wäre auch, die Taschen wie dargestellt sandwichartig zwischen die innere und die äußere Materiallagen 63,64 anzuordnen, welche die Innenseite 3 und die Außenseite 4 der Manschette 10 bilden. Besonders bevorzugt ist es, wenn die Taschen 59 zwischen der äußeren Materiallage 64 und einer im ersten Längsabschnitt 7 auf der äußeren Materiallage 64 angeordneten schlaufenbildenden Zone 47 angebracht sind. Ganz besonders bevorzugt ist es, wenn die Taschen 59 links und rechts neben der fluidleitenden Verbindung zwischen Blase und Druckschlauch (Figuren 1 und 2, in Figur 6 nicht dargestellt) angeordnet sind. Solchenfalls begrenzt der Druckschlauch die Einschiebetiefe der Vorsprünge 62 in die Taschen 59.

Die die Befestigungsmittel 43 bildenden Vorsprünge 62 sind zur Fixierung der Positioniervorrichtung 42 an der Manschette 10 in die dafür vorgesehenen Taschen 59 einschiebbar. Vorteilhafterweise umfassen die Vorsprünge 62 und die Taschen 59 miteinander kooperierende Rastmittel, wie Einrastnasen und korrespondierende Haltemittel, so dass die Vorsprünge in den Taschen 59 in einer vorgegebenen Stellung lösbar gehalten sind.

Denkbar und vorteilhaft wäre auch, das lösbare Festlegen der Positioniervorrichtung 42 an der Manschette alternativ zu den ersten Befestigungsmitteln oder wie dargestellt in Ergänzung dazu durch auf der Innenseite 3 der Positioniervorrichtung 42 vorzusehende Klettbereiche 44 der Positioniervorrichtung, vorzugsweise in Form von Kletthaken, zu ermöglichen, welche mit einer schlaufenbildende Zone 47, vorzugsweise einem Flauschmaterial, auf der Außenseite 4 des ersten Längsabschnitts 7 in Eingriff bringbar sind. Die schlaufenbildende Zone 47 ist überdies vorzugsweise zum Schließen der Manschette mit einem Haftbereich 48 des Endabschnitts der Manschette in Eingriff bringbar.

In Figur 6c ist die an die Manschette 10 lösbar angefügte Positioniervorrichtung 42 dargestellt. Die ersten Befestigungsmittel 43 bildenden Vorsprünge 62 sind in die Taschen 59 eingeschoben. Außerdem haften die Kletthaken umfassenden Klettbereiche der Positioniervorrichtung 44 an der Außenseite 4 der Manschette, welche dort eine entsprechende schlaufenbildende Zone 47 aufweisen, die aus einem Flauschmaterial gebildet sind. Im Ergebnis ist eine erfindungsgemäße Wickelmanschette 10 bereitgestellt, welche an einem ersten Längsabschnitt 7 an einem ersten unteren Randbereich 8 eine Positionierhilfe 13 aufweist, wobei die Positionierhilfe 13 eine Aussparung 14 umfasst, so dass die Manschette passgenau an die Ellenbeuge anlegbar ist und die Gefahr des Verrutschens in Umfangsrichtung der angelegten Manschette reduziert ist.

Mit der lösbar festlegbaren Positioniervorrichtung 42 hat der Benutzer die Wahl, die Manschette gegebenenfalls in wie im Stand der Technik bekannter Weise auch ohne Positionierhilfe zu verwenden.

## Patentansprüche

1. Wickelmanschette (10) für das Messen des Blutdrucks mit einer Längsrichtung (1) und einer Querrichtung (2) und mit einer Innenseite (3) und einer Außenseite (4), wobei die Wickelmanschette (10) einen ersten Längsabschnitt (7) umfasst, und mit einem Endabschnitt (6), der sich in Längsrichtung (1) an den ersten Längsabschnitt (7) anschließt, wobei der erste Längsabschnitt (7) einen ersten unteren Randbereich (8) sowie einen ersten oberen Randbereich (9) aufweist, und wobei die Wickelmanschette (10) einen Blasenbereich (15) umfasst, wobei der Blasenbereich (15) eine Blase (16) aufweist, und wobei der Endabschnitt keine expandierbare Blase aufweist, wobei der erste Längsabschnitt (7) an einem ersten unteren Randbereich (8) eine Positionierhilfe (13) aufweist, wobei die Positionierhilfe (13) eine Aussparung (14) umfasst und wobei die Positionierhilfe (13) einen ersten Flügel (23) und einen zweiten Flügel (24) umfasst und die Aussparung (14) in Längsrichtung (1) beidseitig durch die Flügel (23, 24) begrenzt ist, und wobei der Blasenbereich (15) einen unteren Blasenbereichsrand (17) aufweist und wobei die Aussparung (14) in Querrichtung (2) einen ersten Abstand (18) von 1,5 bis 5,5 cm von dem unteren Blasenbereichsrand (17) aufweist.

2. Wickelmanschette (10) für das Messen des Blutdrucks nach dem vorhergehenden Anspruch, wobei der erste Längsabschnitt (7) mindestens ein Schalenelement (81) aufweist.

3. Wickelmanschette (10) für das Messen des Blutdrucks nach dem vorhergehenden Anspruch, wobei das mindestens eine Schalenelement (81) sich über 50 % - 100 %, insbesondere 65 % bis 98 % und ganz besonders bevorzugt 80 % bis 95 % der Fläche des ersten Längsabschnitts (7) erstreckt.

4. Wickelmanschette (10) für das Messen des Blutdrucks nach einem der vorhergehenden Ansprüche, wobei die Aussparung (14) in Querrichtung (2) einen ersten Abstand (18) von 2,5 bis 4,0 cm und insbesondere von höchsten 3,0 cm von dem unteren Blasenbereichsrand (17) aufweist.

5. Wickelmanschette (10) für das Messen des Blutdrucks nach einem der vorhergehenden Ansprüche, wobei die Aussparung (14) eine im Wesentlichen rechteckige oder trapezförmige oder bogen- oder kurvenförmige Kontur aufweist.

6. Wickelmanschette (10) für das Messen des Blutdrucks nach einem der vorhergehenden Ansprüche, wobei die Positionierhilfe, insbesondere der erste und/oder der zweite Flügel (23,24) ein Versteifungselement (25) aufweist oder davon gebildet wird.

7. Wickelmanschette (10) für das Messen des Blutdrucks nach Anspruch 6, wobei das Versteifungselement (25) durch eine Weitererstreckung des Schalenelements (81) bis in den Bereich der Positionierhilfe (13) hinein gebildet wird.

8. Wickelmanschette (10) für das Messen des Blutdrucks nach Anspruch 6, wobei das Versteifungselement (25) auf der die Innenseite (3) oder der die Außenseite (4) oder zwischen der die Innenseite (3) der Positionierhilfe bildenden Materiallage (63) und der die Außenseite (4) der Positionierhilfe bildenden Materiallage (64) angeordnet ist.

9. Wickelmanschette (10) für das Messen des Blutdrucks nach einem der vorhergehenden Ansprüche 6 oder 8, wobei das Versteifungselement (25) in Querrichtung (2) einen zweiten Abstand (26) von höchstens 6,0 cm, vorzugsweise von höchsten 5,0 cm, insbesondere zwischen 0,25 cm und 4,0 cm, und weiter insbesondere zwischen 0,8 cm und 1,5 cm von dem unteren Blasenbereichsrand (17) aufweist oder sich in Querrichtung (2) über den unteren Blasenbereichsrand (17) hinaus erstreckt.

10. Wickelmanschette (10) für das Messen des Blutdrucks nach einem der vorhergehenden Ansprüche, wobei der erste obere Randbereich (9) eine erste Anlegehilfe (29) mit einem ersten Anfassmittel (30) aufweist.

11. Wickelmanschette (10) für das Messen des Blutdrucks nach Anspruch 10, wobei das erste Anfassmittel (30) einen Greifbereich (31) und einen Markierungsbereich (32) umfasst, wobei der Markierungsbereich (32) eine vom Greifbereich (31) durch Farbe, Material oder Steifigkeit, insbesondere visuell unterscheidbare Greifmarkierung (33) aufweist.

12. Wickelmanschette (10) für das Messen des Blutdrucks nach einem der vorhergehenden Ansprüche, wobei die Wickelmanschette (10) einen Druckschlauch (38) zum Einleiten eines Fluids in die Blase (16) umfasst, wobei an dem Druckschlauch (38) vorzugsweise ein mindestens zweifarbiger Informationsträger (39) angebracht ist.

13. Wickelmanschette (10) für das Messen des Blutdrucks nach einem der vorhergehenden Ansprüche, wobei die Positionierhilfe (13) an einer von der Manschette (10) ablösbaren und wieder festlegbaren Positioniervorrichtung (42) angeordnet ist.

14. Wickelmanschette (10) für das Messen des Blutdrucks nach einem der vorhergehenden Ansprüche, wobei die Innenseite (3) des ersten Längsabschnitts (7) ein Material umfasst oder davon gebildet ist, welches der Innenseite (3) des ersten Längsabschnitts (7) auf der Haut eines Benutzers eine höhere Haftung als eine Außenseite (4) des ersten Längsabschnitts (7) verleiht.

## Claims

1. Wrap cuff (10) for measurement of blood pressure, with a longitudinal direction (1) and a transverse direction (2) and with an inner face (3) and an outer face (4), wherein the wrap cuff (10) comprises a first longitudinal portion (7), and with an end portion (6) which adjoins the first longitudinal portion (7) in the longitudinal direction (1), wherein the first longitudinal portion (7) has a first lower edge region (8) and a first upper edge region (9), and wherein the wrap cuff (10) comprises a bladder region (15), wherein the bladder region (15) has a bladder (16), and wherein the end portion has no expandable bladder, wherein the first longitudinal portion (7) has a positioning aid (13) at a first lower edge region (8), wherein the positioning aid (13) comprises a recess (14), and wherein the positioning aid (13) comprises a first wing (23) and a second wing (24), and the recess (14) is delimited at both ends in the longitudinal direction (1) by the wings (23, 24), and wherein the bladder region (15) has a lower bladder region edge (17), and wherein the recess (14) is at a first distance (18) of 1.5 to 5.5 cm from the lower bladder region edge (17) in the transverse direction (2).

2. Wrap cuff (10) for measurement of blood pressure according to the preceding claim, wherein the first longitudinal portion (7) has at least one shell element (81).

3. Wrap cuff (10) for measurement of blood pressure according to the preceding claim, wherein the at least one shell element (81) extends over 50%-100%, in particular 65% to 98%, very particularly preferably 80% to 95%, of the surface of the first longitudinal portion (7).

4. Wrap cuff (10) for measurement of blood pressure according to one of the preceding claims, wherein the recess (14) is at a first distance (18) of 2.5 to 4.0 cm, in particular of at most 3.0 cm, from the lower bladder region edge (17) in the transverse direction (2).

5. Wrap cuff (10) for measurement of blood pressure according to one of the preceding claims, wherein the recess (14) has a substantially rectangular or trapezoidal or arcuate or curved contour.

6. Wrap cuff (10) for measurement of blood pressure according to one of the preceding claims, wherein the positioning aid, in particular the first and/or second wing (23, 24), has a stiffening element (25) or is formed by same.

7. Wrap cuff (10) for measurement of blood pressure according to Claim 6, wherein the stiffening element (25) is formed by a continuation of the shell element (81) into the region of the positioning aid (13).

8. Wrap cuff (10) for measurement of blood pressure according to Claim 6, wherein the stiffening element (25) is arranged on the material layer (63) forming the inner face (3) or the material layer (64) forming the outer face (4) or between the material layer (63) forming the inner face (3) of the positioning aid and the material layer (64) forming the outer face (4) of the positioning aid.

9. Wrap cuff (10) for measurement of blood pressure according to either of the preceding Claims 6 and 8, wherein the stiffening element (25) is at a second distance (26) of at most 6.0 cm, preferably at most 5.0 cm, in particular between 0.25 and 4.0 cm, and more particularly between 0.8 cm and 1.5 cm, from the lower bladder region edge (17) in the transverse direction (2) or extends beyond the lower bladder region edge (17) in the transverse direction (2).

10. Wrap cuff (10) for measurement of blood pressure according to one of the preceding claims, wherein the first upper edge region (9) has a first fitting aid (29) with a first tactile means (30).

11. Wrap cuff (10) for measurement of blood pressure according to Claim 10, wherein the first tactile means (30) comprises a gripping region (31) and a marking region (32), wherein the marking region (32) has a grip marking (33) that is distinguishable from the gripping region (31) by colour, material or stiffness, in particular visually distinguishable.

12. Wrap cuff (10) for measurement of blood pressure according to one of the preceding claims, wherein the wrap cuff (10) comprises a pressure tube (38) for introducing a fluid into the bladder (16), wherein an at least two-colour information carrier (39) is applied to the pressure tube (38).

13. Wrap cuff (10) for measurement of blood pressure according to one of the preceding claims, wherein the positioning aid (13) is arranged on a positioning device (42) which is detachable from the cuff (10) and is reattachable.

14. Wrap cuff (10) for measurement of blood pressure according to one of the preceding claims, wherein the inner face (3) of the first longitudinal portion (7) comprises or is formed from a material that gives the inner face (3) of the first longitudinal portion (7) greater adhesion to the skin of a user than an outer face (4) of the first longitudinal portion (7).

## Revendications

1. Brassard (10) pour la mesure de la tension artérielle, doté d'un sens longitudinal (1) et d'un sens transversal (2) et d'une face intérieure (3) et d'une face extérieure (4), ce brassard (10) comprenant une première section longitudinale (7), et une section terminale (6) qui se raccorde dans le sens longitudinal (1) à la première section longitudinale (7), la première section longitudinale (7) présentant une première zone périphérique inférieure (8) et une première zone périphérique supérieure (9), et le brassard (10) comprenant une zone à poche (15), la zone à poche (15) présentant une poche (16), et la section terminale ne présentant pas de poche dilatable, la première section longitudinale (7) présentant au niveau d'une première zone périphérique inférieure (8) une aide au positionnement (13), l'aide au positionnement (13) comprenant un évidement (14) et l'aide au positionnement (13) comprenant une première aile (23) et une seconde aile (24) et l'évidement (14) étant limité des deux côtés par les ailes (23, 24) dans la direction longitudinale (1), et la zone à poche (15) présentant un bord inférieur de partie à poche (17) et l'évidement (14) présentant dans le sens transversal (2) un premier espacement (18) de 1, 5 à 5, 5 cm par rapport au bord inférieur de partie à poche (17).

2. Brassard (10) pour la mesure de la tension artérielle selon la revendication précédente, dans lequel la première section longitudinale (7) présente au moins un élément à coque (81).

3. Brassard (10) pour la mesure de la tension artérielle selon la revendication précédente, dans lequel l'au moins un élément à coque (81) s'étend sur 50 % à 100 %, en particulier 65 % à 98 % et très préférentiellement 80 % à 95 % de la surface de la première section longitudinale (7).

4. Brassard (10) pour la mesure de la tension artérielle selon une des revendications précédentes, dans lequel l'évidement (14) présente dans le sens transversal (2) un premier espacement (18) de 2, 5 à 4, 0 cm et en particulier au maximum de 3, 0 cm par rapport au bord de la partie à poche (17).

5. Brassard (10) pour la mesure de la tension artérielle selon une des revendications précédentes, dans lequel l'évidement (14) présente un contour sensiblement rectangulaire ou trapézoïdal ou de forme arquée ou courbée.

6. Brassard (10) pour la mesure de la tension artérielle selon une des revendications précédentes, dans lequel l'aide au positionnement, en particulier la première et/ou la seconde aile (23, 24), présente un élément de raidissement (25) ou en est constituée.

7. Brassard (10) pour la mesure de la tension artérielle selon la revendication 6, dans lequel l'élément de raidissement (25) est constitué par une suite de l'extension de l'élément à coque (81) jusque dans la zone de l'aide au positionnement (13).

8. Brassard (10) pour la mesure de la tension artérielle selon la revendication 6, dans lequel l'élément de raidissement (25) est disposé sur la couche de matériau (63) formant la face intérieure (3) ou la face extérieure (4) ou entre celle formant la face intérieure (3) de l'aide au positionnement et la couche de matériau (64) formant la face extérieure (4) de l'aide au positionnement.

9. Brassard (10) pour la mesure de la tension artérielle selon une des revendications précédentes 6 ou 8, dans lequel l'élément de raidissement (25) présente dans le sens transversal (2) un second espacement (26) valant au maximum 6, 0 cm, de préférence au maximum 5, 0 cm, en particulier situé entre 0, 25 cm et 4, 0 cm et plus particulièrement entre 0, 8 cm et 1, 5 cm, par rapport au bord inférieur de la partie à poche (17) ou s'étend dans le sens transversal (2) au-delà du bord inférieur de la partie à poche (17).

10. Brassard (10) pour la mesure de la tension artérielle selon une des revendications précédentes, dans lequel la première zone périphérique supérieure (9) présente une première aide à la pose (29) comportant un premier moyen de saisie (30).

11. Brassard (10) pour la mesure de la tension artérielle selon la revendication 10, dans lequel le premier moyen de saisie (30) comprend une zone de préhension (31) et une zone de marquage (32), la zone de marquage (32) présentant un marquage de préhension (33) pouvant être différencié de la zone de préhension (31) en particulier visuellement par sa couleur, sa matière ou sa rigidité.

12. Brassard (10) pour la mesure de la tension artérielle selon une des revendications précédentes, le brassard (10) comprenant un tuyau sous pression (38) pour introduire un fluide dans la poche (16), un support d'information (39) au moins bicolore étant de préférence installé au niveau du tuyau sous pression (38).

13. Brassard (10) pour la mesure de la tension artérielle selon une des revendications précédentes, dans lequel l'aide au positionnement (13) est disposée au niveau d'un dispositif de positionnement (42) détachable du brassard (10) et pouvant être replacé dessus.

14. Brassard (10) pour la mesure de la tension artérielle selon une des revendications précédentes, dans lequel la face intérieure (3) de la première section longitudinale (7) comprend un matériau ou est composée d'un matériau qui confère à la face intérieure (3) de la première section longitudinale (7), sur la peau d'un utilisateur, une plus forte adhérence qu'une face extérieure (4) de la première section longitudinale (7).
